# EUROPEAN PATENT SPECIFICATION

(11) **EP 2 895 119 B1**
(45) Date of publication and mention of the grant of the patent: **25.10.2017**
(21) Application number: 13837423.6
(22) Date of filing: 17.09.2013
(51) Int. Cl.: A61F 5/01

(54) **ANKLE BRACE**
FUSSGELENKSTÜTZE
ATTELLE POUR CHEVILLE

(30) Priority: 17.09.2012 US 201261702081 P
(43) Date of publication of application: 22.07.2015
(73) Proprietor: Shock Doctor Inc., Minnetonka, Minnesota 55305 (US)
(72) Inventor: BEST, William, Orono, Minnesota 55356 (US); JOURDE, Bastien, Montreal, Québec H2W 2J5 (CA); PETELLE, Thierry, Montreal, Québec H2S 2C8 (CA)
(74) Representative: Pfenning, Meinig & Partner mbB
(86) International application number: PCT/US2013/060152
(87) International publication number: WO 2014/043695

(56) References cited:
- US-A- 5 031 607
- US-A- 5 429 588
- US-A- 5 429 588
- US-A- 5 496 263
- US-A- 5 971 946
- US-A1- 2009 247 920
- US-A1- 2010 137 770
- US-B1- 6 602 215
- US-B1- 7 364 561

## Description

### Technical Field

The disclosure pertains generally to protective and rehabilitative gear and more particularly to an ankle brace.

### Background

Each year, many people, both athletes and non-athletes, suffer ankle injuries. In some cases, athletes wrap their ankles with adhesive tape in an attempt to prevent ankle injuries and/or to support their ankles after an injury has occurred. In many instances, athletes and others use ankle braces to protect and/or to rehabilitate their ankles.

US 5 031 607 A describes an ankle brace, for either the left or right foot, for protecting an exercising an injured ankle that includes a heel portion, inner and outer pivot legs, and padding which lies between the wearer's leg and the brace.

US 7 364 561 B1 discloses an ankle brace with a removable plate comprising a stirrup having a bottom portion and an inner and outer upright leg depending upward thereform. An inner pivot leg is pivotally attached to an upper end of the inner upright leg of the stirrup and an outer pivot leg is pivotally attached to an upper end of the outer upright leg of the stirrup. An exterior plate is removably attached to an outer side of the outer pivot leg. The ankle brace further comprises one or more horizontal straps extending through an elongate slot formed by the exterior plate and securing together an intermediate portion of the ankle brace. US 5 971 946 A refers to a reversible ankle brace including a foot plate and opposing uprights extending upward from opposite sides of the foot plate. A lateral side panel is rotatably connected to the one upright, and a medical side panel is rotatably connected to the other upright at a relatively greater distance away from the foot plate.

US 5 496 263 A shows an ankle brace with an upper calf member hingedly connected to a lower foot member, and a plurality of reinforced areas for strengthening and supporting a region around the talus bone. The reinforced areas are located above and below the hinge, or in the proximate vicinity of the ankle and talus bone. This brace also comprises two horizontal straps extending through slots formed in the outer surface of the upper calf members and securing together the intermediate and upper portions of the ankle brace. US 2010/137770 A1 discloses an ankle brace including a calf body defining lateral and medial frontal sides spaced by a frontal opening, and first and second straps depending from the first and second frontal sides of the calfbody, respectively. The first and second straps are arranged to extend juxtaposed across the opening and secure to the second and first frontal sides, respectively.

US 5 429 588 A1 describes an ankle foot orthosis has a foot platform base comprising a rigid chassis. The orthosis is provided with attachment hinges for securing a pair of upright side-members to the foot platform base. The side-members are covered with hook and loop attachment material which releasably secures them to a wrap which encloses the foot, ankle and lower leg. The orthosis is secured to a patient with a series of straps.

While a variety of ankle braces are known, there is a desire for continued improvement in the performance and comfort of known ankle braces. There is a desire for an ankle brace that is easy to use and comfortable for the wearer.

### Summary

To achieve this purpose, the present invention provides a reversible ankle brace comprising the features of claim 1. Preferred embodiments of this ankle brace are defined in the dependent claims.

The invention pertains to an ankle brace that is easy to use, is comfortable for the wearer and is reversible, i.e., a single ankle brace may be used on either the wearer's left foot or right foot, depending on where the injury is. A reversible ankle brace means that a wearer can use the brace again, even if, for example, they injure their other ankle.

Accordingly, a reversible ankle brace according to the invention includes a stirrup having a base plate and first and second stirrup arms. A first sideplate is secured to the first stirrup arm at a first pivot point and a second sideplate is secured to the second stirrup arm at a second pivot point. First and second elongate slots are formed in the outer surfaces of the first and second sideplates. A first pad is releasably securable to an inner surface of the first sideplate and a second pad is releasably securable to an inner surface of the second sideplate. A first horizontal strap is releasably securable about an exterior of the first pad and the second pad and secures an upper portion of the ankle brace. A second horizontal strap extends through the first and second elongate slots and secures together an intermediate portion of the ankle brace.

The reversible ankle brace further includes a cross strap. The cross strap is configured to fit through a pair of slots in the base plate, cross a first time on a back side of the ankle brace and cross a second time on a front side of the ankle brace. In some instances, the cross strap has a first end and a second end, and the first and second ends are adapted to be releasably securable to the first horizontal strap after crossing the first time behind the ankle brace and crossing the second time in front of the ankle brace.

The present disclosure also describes a method of adjusting and putting a reversible ankle brace according to the invention on a wearer. The method includes selecting a pair of slots that extend through a base plate of the reversible ankle brace in accordance with whether the ankle brace will be used on the wearer's left foot or their right foot. A cross strap is extended through the selected pair of slots such that the cross strap has a first end extending from a first side of the base plate and a second end extending from a second side of the base plate. After the wearer's foot is positioned within the reversible ankle brace, a first horizontal strap that extends around an exterior of the reversible ankle brace is secured in place in order to secure together an upper portion of the reversible ankle brace. A second horizontal strap that extends around an exterior of the reversible ankle brace is secured in place in order to secure together an intermediate portion of the reversible ankle brace. The first and second ends of the cross strap are extended inside the first and second side pads, the cross strap passing over either side of the wearer's foot and then wrapping behind the wearer's calf, the first and second ends of the cross strap crossing each other proximate the wearer's calf. The first and second ends of the cross strap are wrapped in front of the wearer's shin, the first and second ends of the cross strap crossing each other again proximate the wearer's shin. The first and second ends of the cross strap are secured to either side of one of the first and second horizontal straps.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### Brief Description of the Figures

Figure 1 is a perspective view of an ankle brace shown on a foot.
Figure 2 is a perspective view of the ankle brace of Figure 1, shown without the foot.
Figure 3 is a front perspective view of an ankle brace in accordance with embodiments of the invention, shown on a foot.
Figure 4 is a front perspective view of the ankle brace of Figure 3, shown without the foot.
Figure 5 is a rear perspective view of the ankle brace of Figure 3, shown without the foot.
Figure 6 is a front perspective view of a portion of the ankle brace of Figure 1, shown with side pads attached.
Figure 7 is a front view of the ankle brace of Figure 6.
Figure 8 is a front perspective view of a portion of the ankle brace of Figure 7, shown without side pads.
Figure 9 is a front view of the ankle brace of Figure 8.
Figure 10 is a medial side view of the ankle brace of Figure 6.
Figure 11 is a lateral side view of the ankle brace of Figure 6.
Figure 12 is an exploded schematic view of the ankle brace of Figure 1.
Figure 13 is an exploded schematic view of the ankle brace of Figure 3.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### Detailed Description

Various embodiments relate to a reversible ankle brace that is easy to use, is comfortable for the wearer and is reversible. Accordingly, a wearer may use the inventive ankle brace on either their left foot, to support their left ankle, or their right foot, to support their right ankle, without having to purchase more than one ankle brace. Though ankle braces according to various embodiments include the features and/or achieve the foregoing advantages, alternative or additional features and advantages are contemplated.

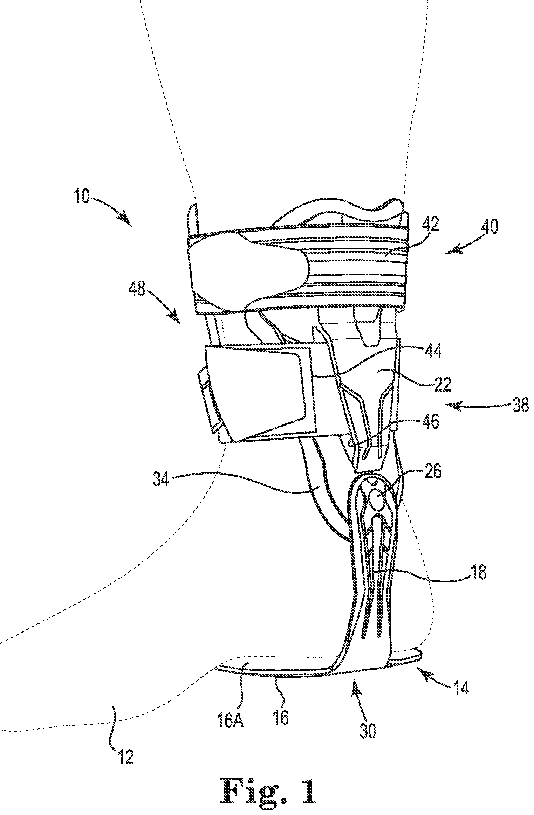

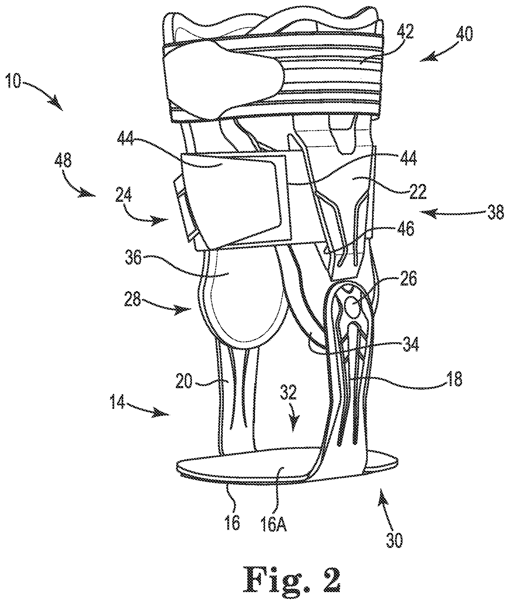

Figures 1 and 2 provide perspective views of an exemplary ankle brace 10, not in accordance with the present invention, but described for illustrative purposes. In Figure 1, the ankle brace 10 is shown on a wearer's foot 12 while Figure 2 provides a similar view without the foot 12, in order to show additional details of the ankle brace 10.

The ankle brace 10 includes a stirrup 14 having a base plate 16, a first stirrup arm 18 and a second stirrup arm 20. As illustrated, the first stirrup arm 18 is approximately centered along a first side 30 of the stirrup 14 and the second stirrup arm 20 is approximately centered along a second side 32 of the stirrup 14. In some embodiments, centering the first stirrup arm 18 and the second stirrup arm 20 facilitates being able to reverse the ankle brace 10 for use on either a left ankle or a right ankle. Optionally, the stirrup 14 is integrally molded as a unitary piece, including the base plate 16, the first stirrup arm 18 and the second stirrup arm 20. The stirrup 14 is optionally formed of a relatively rigid polymeric material. In some embodiments, a soft foam pad 16A is disposed over the base plate 16 for the wearer's comfort.

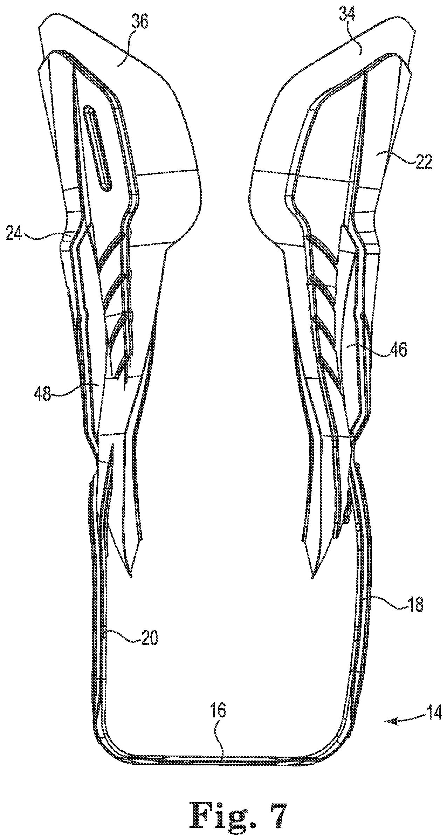

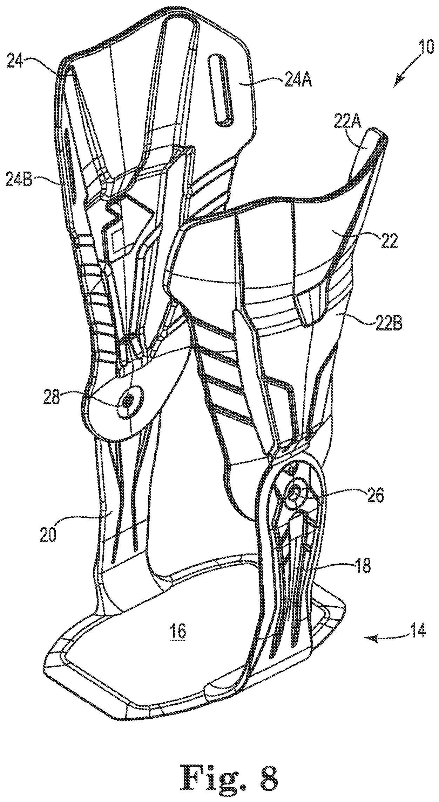

In some embodiments, the ankle brace 10 is configured to wear with one of the first stirrup arm 18 and the second stirrup arm 20 on the medial (inside) side of the wearer's ankle and have the other of the first stirrup arm 18 and the second stirrup arm 20 on the lateral (outside) side of the wearer's ankle. In some instances, one of the first stirrup arm 18 and the second stirrup arm 20 has a slightly different length or profile than the other. For example, and with particular reference to Figures 7 and 8, the first stirrup arm 18 is longer than the second stirrup arm 20 and has a more pronounced curve. The second stirrup arm 20 is straighter than the first stirrup arm 18, and has a smaller curve near a top thereof.

First and second sideplates 22 and 24 are attached to the first and second stirrup arms 18, 20, respectively, at a first pivot point 26 and a second pivot point 28. In some embodiments, the stirrup 14 is symmetric about a medial, vertical plane (not illustrated, for clarity) that extends through the first pivot point 26 and the second pivot point 28 and that is perpendicular to the base plate 16. It will be appreciated that the first sideplate 22 may be permitted to pivot forwards and backwards relative to the first stirrup arm 18 while being constrained against relative movement in a side to side fashion. Similarly, the second sideplate 24 may be permitted to pivot forwards and backwards relative to the second stirrup arm 20 while being constrained against relative movement in a side to side fashion. The first sideplate 22 and the second sideplate 24 are optionally formed of a relatively rigid polymeric material.

A first pad 34 is releasably securable to the first sideplate 22 and a second pad 36 is releasably securable to the second sideplate 24. In some instances, a hook-and-loop fastening system, commercially known as VELCRO, is used to secure the first pad 34 to the first sideplate 22 and to secure the second pad 36 to the second sideplate 24. Optionally, the first and second pads 34, 36 are secured using alternate fashioning techniques such as snaps or pressure sensitive adhesives. The first pad 34 and the second pad 36 are optionally formed of a soft polymeric material such as a foam.

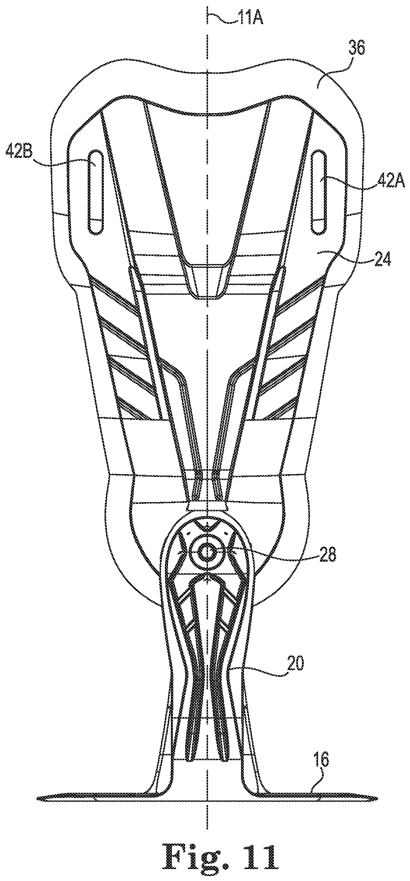

In some instances, the ankle brace 10 includes an intermediate portion 38 and an upper portion 40. As illustrated, a first horizontal strap 42 extends around an exterior of the upper portion 40 of the ankle brace 10. It will be appreciated that the first horizontal strap 42, if appropriately tightened up, will secure together the upper portion 40 of the ankle brace 10. In some cases (see Figure 11) the first horizontal strap 42 is releasably secured to either the first sideplate 22 or the second sideplate 24 in order to appropriately locate the first horizontal strap 42 relative to the ankle brace 10. The first horizontal strap 42 is dimensioned to fit at least once around the upper portion 40 of the ankle brace 10. Optionally, the first horizontal strap 42 is dimensioned to fit more than once around the upper portion 40 of the ankle brace 10. The first horizontal strap 42 is configured to be securable to itself, such as by using hook-and-loop fasteners and may be formed of any elastomeric material.

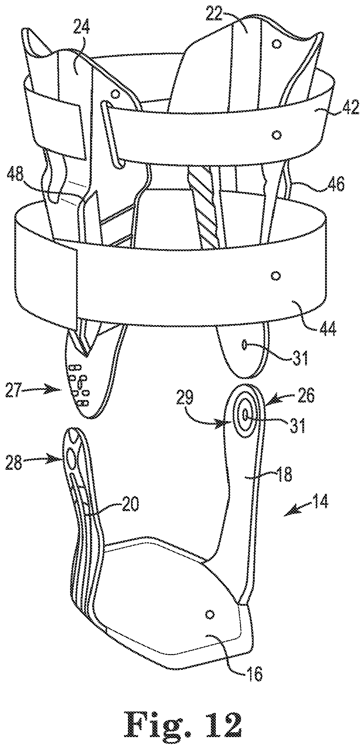

A second horizontal strap 44 extends around an exterior of the intermediate portion 38 of the ankle brace 10. As illustrated, the first sideplate 22 includes a first elongate slot 46. The second sideplate 24 includes a second elongate slot 48 (visible in Figure 12). In some instances, the second horizontal strap 44 is extended through the first elongate slot 46 and the second elongate slot 48 in order to help guide the placement of the second horizontal strap 44. Optionally, in the ankle brace in accordance with the invention the first and second elongate slots 46, 48 also provide guidance to a cross strap, as will be discussed with respect to subsequent Figures.

Optionally, the second horizontal strap 44 may be releasably secured (instead of or in addition to the first and second elongate slots 46, 48) to either the first sideplate 22 and the second side plate 24 in order to appropriately locate the second horizontal strap 44 relative to the ankle brace 10. The second horizontal strap 44 is dimensioned to fit at least once around the intermediate portion 38 of the ankle brace 10. Optionally, the second horizontal strap 44 is dimensioned to fit more than once around the intermediate portion 38 of the ankle brace 10. The second horizontal strap 44 is configured to be securable to itself, such as by using hook-and-loop fasteners and may be formed of any elastomeric material.

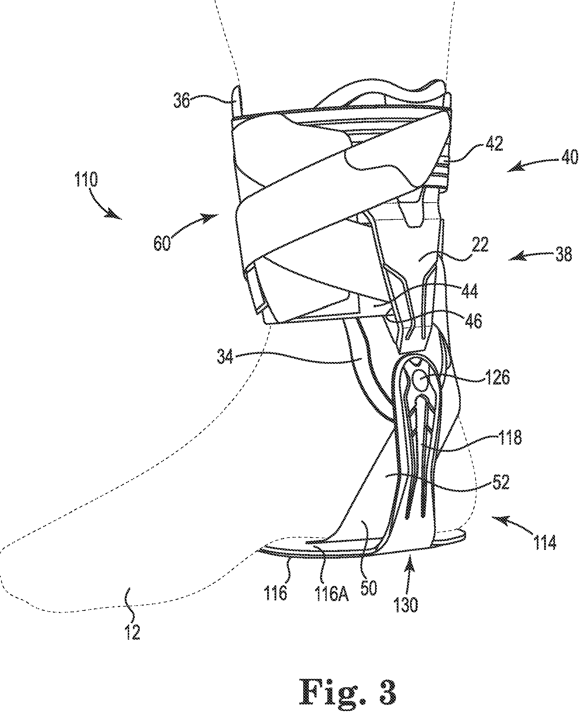

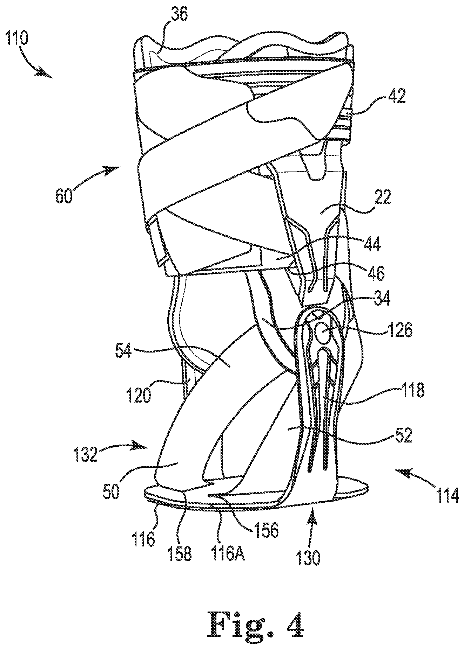

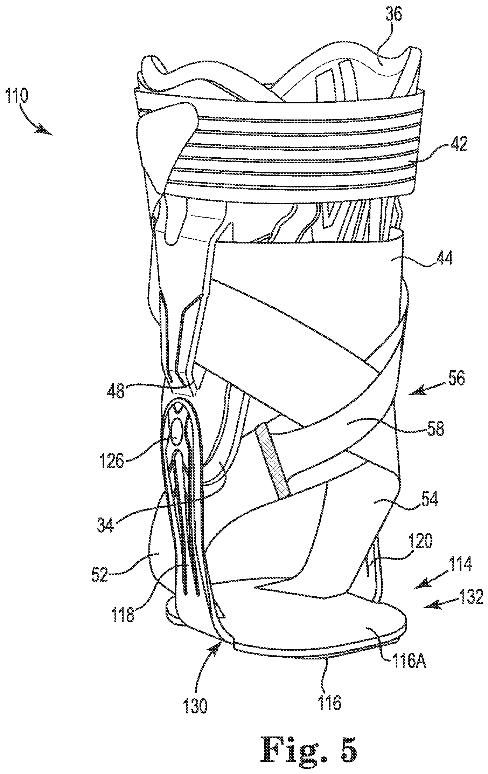

Figures 3, 4 and 5 provides prospective views of an ankle brace 110 in accordance with the present invention. In Figure 3, the ankle brace 110 is shown on a wearer's foot 12 while Figures 4 and 5 provide a similar view without the foot 12, in order to show additional details of the ankle brace 110. According to some embodiments, the ankle brace 110 shares some common features with the ankle brace 10, and similar reference numbers are used to denote similar features. Various features that are the same between the ankle brace 10 and the ankle brace 110 are not be described again with specific reference to the ankle brace 110.

The ankle brace 110 according to the invention primarily differs from the ankle brace 10 by including a cross strap 50. The ankle brace 110 includes a stirrup 114 that is configured to accommodate the cross strap 50. The stirrup 114 has a base plate 116, a first stirrup arm 118 and a second stirrup arm 120. As illustrated, the first stirrup arm 118 is approximately centered along a first side 130 of the stirrup 114 and the second stirrup arm 120 is approximately centered along a second side 132 of the stirrup 14. Optionally, the stirrup 114 is integrally molded as a unitary piece, including the base plate 116, the first stirrup arm 118 and the second stirrup arm 120. In some embodiments, a soft foam pad 116A is disposed over the base plate 116 for the wearer's comfort, or other purposes as desired.

First and second sideplates 22 and 24 are attached to the first and second stirrup arms 118, 120, respectively, at a first pivot point 126 and a second pivot point 128. In some embodiments, the stirrup 114 is symmetric about a medial, vertical plane (not illustrated) that extends through the first pivot point 126 and the second pivot point 128 and that is perpendicular to the base plate 116. It will be appreciated that the first sideplate 22 may be permitted to pivot forwards and backwards relative to the first stirrup arm 118 while being constrained against relative movement in a side to side fashion. Similarly, the second sideplate 24 may be permitted to pivot forwards and backwards relative to the second stirrup arm 120 while being constrained against relative movement in a side to side fashion.

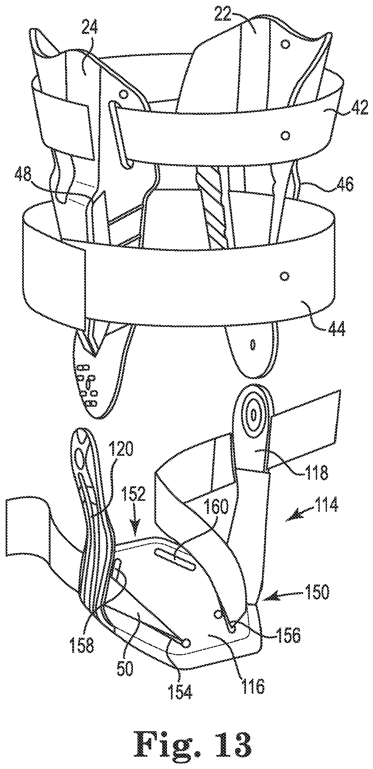

The base plate 116 includes a first pair 150 of slots and a second pair 152 (visible in Figure 13) of slots. The first pair 150 of slots includes a slot 154 and a slot 156. The second pair 152 of slots includes a slot 158 and a slot 160. It will be appreciated that a wearer may select between the first pair 150 of slots and the second pair 152 of slots depending on whether they desire to use the ankle brace 110 on their left ankle or their right ankle. For example, when worn on the right ankle, typically the first pair of slots 150 is selected.

In some embodiments, the wearer may prefer to have one of the first stirrup arm 118 and the second stirrup arm 120 on the medial (inside) side of their ankle and have the other of the first stirrup arm 118 and the second stirrup arm 120 on the lateral (outside) side of their ankle. In some instances, one of the first stirrup arm 118 and the second stirrup arm 120 may have a slightly different length or profile than the other, where the first stirrup arm 118 would typically be worn on the inside of the ankle.

Optionally, the cross strap 50 is used to provide improved heel retention within the ankle brace 110. The cross strap 50 may be formed of any elastomeric material. In some instances, the cross strap 50 is a single strap, i.e., the first end 52 and the second end 54 are part of a unitary cross strap 50. The cross strap 50 extends from either the first pair 150 of slots or the second pair 152 of slots, and may be considered as being divided into a first end 52 and a second end 54. As illustrated in Figures 3 to 5, the first end 52 and the second end 54 extend up from the base plate 116 and pass inside the first stirrup arm 118 and the second stirrup arm 120, respectively, and wrap along either side of the wearer's foot 12.

As shown in Figure 5, the first end 52 and the second end 54 of the cross strap 50 intersect at a position 56 that would be behind the wearer's calf. Optionally, the first end 52 includes a slot 58 through which the second end 54 may pass. It will be appreciated that the second end 54 is free to move with respect to the first end 52 at the position 56. The cross strap 50 can be further wrapped such that the first end 52 passes through the second elongate slot 48 and the second end 54 passes through the first elongate slot 46 (not visible in Figure 5). The cross strap 50 can be further wrapped such that the first end 52 and the second end 54 intersect at a position 60 that would be adjacent the wearer's shin. The second end 54 is free to move with respect to the first end 52 at the position 60. Optionally, the first end 52 and the second end 54 continue on and are releasably secured to the first horizontal band 42.

In an illustrative embodiment, and in order to adjust and wear the ankle brace 110, a user may select a pair 150, 152 of slots within the base plate 116, depending on which foot/ankle they wish to use the ankle brace 110 with. The wearer can extend the cross strap 50 through the selected pair 150, 152 of slots such that the cross strap 50 has a first end 52 extending from a first side of the base plate 116 and a second end 54 extending from a second side of the base plate 116.

The wearer would then place their foot within the ankle brace 110, and then would secure the first horizontal strap 42 that extends around an exterior of the ankle brace 110 in order to secure together an upper portion 40 of the ankle brace 110. The wearer would then secure the second horizontal strap 44 that extends around an exterior of the ankle brace 110 in order to secure together an intermediate portion 38 of the ankle brace 110. It will be appreciated that the first and second horizontal straps 42, 44 may be secured in place in either order, i.e., in some cases, the wearer may decide to secure the second horizontal strap 44 in place before securing in place the first horizontal strap 42.

Next, the wearer would extend the first and second ends 52, 54 of the cross strap 50 inside the first and second side pads 34, 36, the cross strap 50 passing over either side of the wearer's foot 12 and then wrapping behind the wearer's calf such that the first and second ends 52, 54 of the cross strap cross each other proximate the wearer's calf. The wearer would then wrap the first and second ends 52, 54 of the cross strap 50 in front of the wearer's shin such that the first and second ends 52, 54 of the cross strap 50 cross each other again proximate the wearer's shin. The wearer would then secure the first and second ends 52, 54 of the cross strap 50 to either side of one of the first and second horizontal straps 42, 44.

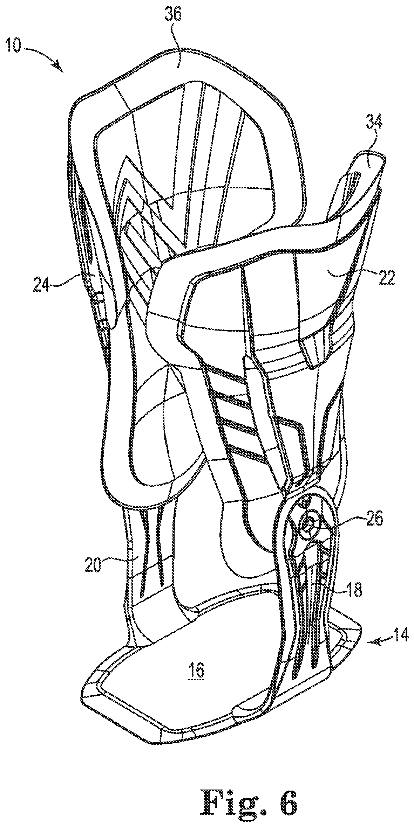

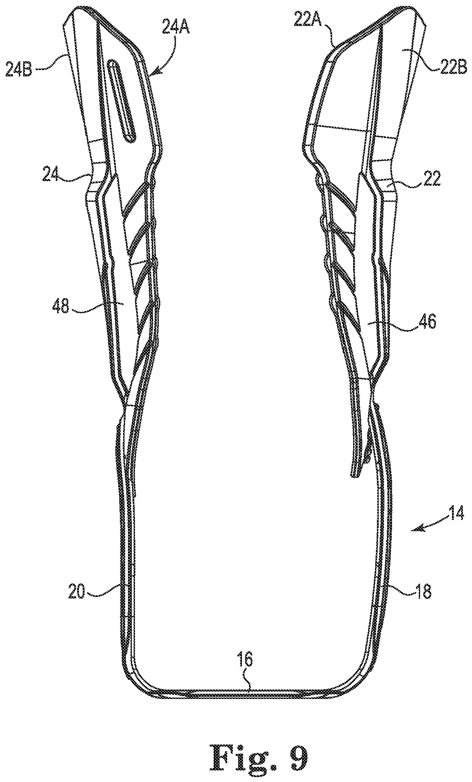

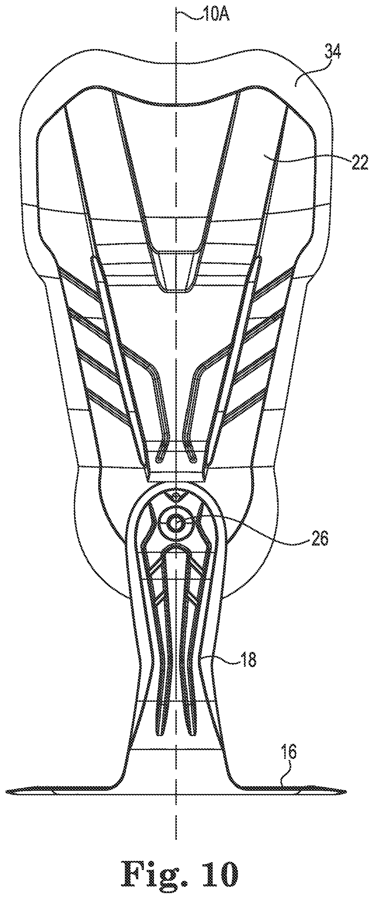

Figures 6 through 11 provide further views of elements of the ankle brace 10 already discussed. In particular, Figure 6 is a front perspective view of a portion of the ankle brace of Figure 1, shown with side pads attached, while Figure 7 is a front view of the ankle brace of Figure 6. Figure 8 is a front perspective view of a portion of the ankle brace of Figure 7, shown without side pads. Figure 9 is a front view of the ankle brace of Figure 8. Figure 10 is a medial side view of the ankle brace of Figure 6 and Figure 11 is a lateral side view of the ankle brace of Figure 6. It will be appreciated that the views of ankle brace 10 provided in Figures 6 through 11 are, with the exception of the stirrup, substantially similar to the ankle brace 110.

With reference to Figure 8, it can be seen that the first sideplate 22 includes an inner surface 22A and an outer surface 22B and that the second sideplate 24 includes an inner surface 24A and an outer surface 24B. The first pad 34 may be releasably securable to the inner surface 22A of the first sideplate 22. The second pad 36 may be releasably securable to the inner surface 24A of the second sideplate 24. The first elongate slot 46 may be formed within the outer surface 22B of the first sideplate 22 and the second elongate slot 48 may be formed within the outer surface 24B of the second sideplate 22.

Figure 10 is a medial side view of the ankle brace 10 and illustrates that the first stirrup arm 18 is centrally disposed along a side of the base plate 16 and that the first sideplate 22 is symmetric about a vertical midline 10A. Figure 11 is a lateral side view of the ankle brace 10 and illustrates that the second stirrup arm 20 is centrally located along a side of the base plate 16 and further illustrates that the second sideplate 22 is symmetric about a vertical mid-line 11A. Figure 11 also illustrates that the second sideplate 22 optionally includes slots 42A and 42B. The slots 42A and 42B, if present, are used to releasably secure the first horizontal strap 42 in place relative to the second sideplate 22 in order to facilitate wrapping the first horizontal strap 42 around the ankle brace 10.

Figures 12 and 13 are schematic illustrations of portions of the ankle brace 10 and the ankle brace 110, respectively, and provide further details regarding the first pivot point 26 and the second pivot point 28. While Figures 12 and 13 only show the outer surface 24B of the second sideplate 24 and the inner surface 22A of the first sideplate 22, it will be appreciated that these elements are symmetric, and thus features shown on the outer surface 24B of the second sideplate 22 are duplicated on the outer surface 22B of the first sideplate 22, and features shown on the inner surface 22A of the first sideplate 22 are duplicated on the inner surface 24A of the second sideplate 24.

Each sideplate 22, 24 includes a series of protrusions 27 that fit into a corresponding circular groove 29 that is formed into each stirrup arm 18, 20 (and 118, 120). It will be appreciated that the combination of protrusions 27 on the sideplates 22, 24 and corresponding circular grooves 29 on the stirrup arms 18, 20 facilitate and guide relative pivoting between the sideplate 22, 24 and the stirrup arm 18, 20. Each pivot point 26, 28 also includes an aperture 31 through which a rivet or similar fastener may be disposed.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the following claims.

## Claims

1. A reversible ankle brace (110) adapted for reversible wear on a left ankle or a right ankle, the ankle brace (110) comprising:
a stirrup (114) including a base plate (116), a first stirrup arm (118) and a second stirrup arm (120);
a first sideplate (22) secured to the first stirrup arm (118) at a first pivot point (126), the first sideplate (22) including a first inner surface (22A) and a first outer surface (22B), a first elongate slot (46) formed in the first outer surface (22B);
a second sideplate (24) secured to the second stirrup arm (120) at a second pivot point (128), the second sideplate (24) including a second inner surface (24A) and a second outer surface (24B), a second elongate slot (48) formed in the second outer surface (24B);
a first pad (34) releasably securable to the first inner surface (22A);
a second pad (36) releasably securable to the second inner surface (24A);
a first horizontal strap (42) releasably securable about an exterior of the first pad (34) and the second pad (36), the first horizontal strap (42) securing together an upper portion of the ankle brace (110);
a second horizontal strap (44) extending through the first elongate slot (46) and the second elongate slot (48), the second horizontal strap (44) releasably securable to itself, the second horizontal strap (44) securing together an intermediate portion (38) of the ankle brace (110); and
a third strap member (50)
wherein the base plate (116) includes a first pair (150) of slots (154, 156) for accommodating the third strap member (50) when the ankle brace (110) is configured for use on a right ankle and a second pair (152) of slots (158, 160) for accommodating the third strap member (50) when the ankle brace (110) is configured for use on a left ankle.

2. The reversible ankle brace of claim 1, wherein the stirrup (114) has a first side and a second side, the first stirrup arm (118) being approximately centered along the first side and the second stirrup arm (120) being approximately centered along the second side.

3. The reversible ankle brace of claim 1 or claim 2, wherein the base plate (116), the first stirrup arm (118) and the second stirrup arm (120) are integrally molded as a unitary piece.

4. The reversible ankle brace of any of claims 1 to 3, further comprising a foam pad (16A) adapted to fit onto the base plate (116).

5. The reversible ankle brace of claim 1 , wherein the third strap member (50) is configured to fit through one of the pair of slots, cross a first time on a back side of the ankle brace (110) and cross a second time on a front side of the ankle brace (110).

6. The reversible ankle brace of claim 1, wherein the third strap member (50) has a first end (52) and a second end (54), and the first (52) and second (54) ends are adapted to be releasably securable to the first horizontal strap (42) after crossing the first time behind the ankle brace (110) and crossing the second time in front of the ankle brace (110).

7. The reversible ankle brace of any of claims 1 to 6, wherein the first sideplate (22) is secured to the first stirrup arm (118) at the first pivot point (126) such that the first sideplate (22) can pivot towards a front of the stirrup (114) and towards a back of the stirrup (114) but is constrained from moving side to side.

8. The reversible ankle brace of any of claims 1 to 7, wherein the second sideplate (24) is secured to the second stirrup arm (120) at the second pivot point (128) such that the second sideplate (24) can pivot towards a front of the stirrup (114) and towards a back of the stirrup (114) but is constrained from moving side to side.

9. The reversible ankle brace of any of claims 1 to 8, wherein the first sideplate (22) is symmetric about a vertical mid-line (10A, 11A).

10. The reversible ankle brace of any of claims 1 to 9, wherein the second sideplate (24) is symmetric about a vertical mid-line (10A, 11A).

11. The reversible ankle brace of any of claims 1 to 10, wherein the stirrup (114) is symmetric about a plane that is drawn through the first pivot point (126) and the second pivot point (128) and that is perpendicular to the base plate (116).

## Patentansprüche

1. Doppelseitige Knöchelstütze (110), die ausgestaltet ist zum doppelseitigen Tragen auf einem linken Knöchel oder einem rechten Knöchel, welche Knöchelstütze (110) aufweist:
einen Bügel (114), enthaltend eine Basisplatte (116), einen ersten Bügelarm (118) und einen zweiten Bügelarm (120);
eine erste Seitenplatte (22), die an dem ersten Bügelarm (118) an einem ersten Schwenkpunkt (126) befestigt ist,
wobei die erste Seitenplatte (22) eine erste innere Oberfläche (22A) und eine erste äußere Oberfläche (22B) enthält und ein erster länglicher Schlitz (46) in der ersten äußeren Oberfläche (22B) gebildet ist;
eine zweite Seitenplatte (24), die an dem zweiten Bügelarm (120) an einem zweiten Schwenkpunkt (128) befestigt ist, wobei die zweite Seitenplatte (24) eine zweite innere Oberfläche (24A) und eine zweite äußere Oberfläche (24B) enthält und ein zweiter länglicher Schlitz (48) in der zweiten äußeren Oberfläche (24B) gebildet ist;
ein erstes Polster (34), das lösbar an der ersten inneren Oberfläche (22A) befestigbar ist;
ein zweites Polster (36), das lösbar an der zweiten inneren Oberfläche (24A) befestigbar ist;
einen ersten horizontalen Riemen (42), der lösbar um ein Äußeres des ersten Polsters (34) und des zweiten Polsters (36) befestigbar ist, wobei der erste horizontale Riemen (42) einen oberen Bereich der Knöchelstütze (110) zusammenhält;
einen zweiten horizontalen Riemen (44), der sich durch den ersten länglichen Schlitz (46) und den zweiten länglichen Schlitz (48) erstreckt, wobei der zweite horizontale Riemen (44) lösbar an sich selbst befestigbar ist und der zweite horizontale Riemen (44) einen Mittelbereich (38) der Knöchelstütze (110) zusammenhält; und
ein drittes Riementeil (50),
wobei die Basisplatte (116) ein erstes Paar (150) von Schlitzen (154, 156) zum Aufnehmen des ersten Riementeils (50), wenn die Knöchelstütze (110) zur Verwendung auf einem rechten Knöchel konfiguriert ist, und ein zweites Paar (152) von Schlitzen (158, 160) zur Aufnahme des dritten Riementeils (50), wenn die Knöchelstütze (110) zur Verwendung auf einem linken Knöchel konfiguriert ist, enthält.

2. Doppelseitige Knöchelstütze nach Anspruch 1, bei der der Bügel (114) eine erste Seite und eine zweite Seite hat, wobei der erste Bügelarm (118) angenähert in der Mitte entlang der ersten Seite angeordnet ist und der zweite Bügelarm (120) angenähert in der Mitte entlang der zweiten Seite angeordnet ist.

3. Doppelseitige Knöchelstütze nach Anspruch 1 oder Anspruch 2, bei der die Basisplatte (116), der erste Bügelarm (118) und der zweite Bügelarm (120) integrierend als ein einheitliches Stück geformt sind.

4. Doppelseitige Knöchelstütze nach einem der Ansprüche 1 bis 3, weiterhin aufweisend ein Schaumpolster (16A), das ausgestaltet ist, auf die Basisplatte (116) zu passen.

5. Doppelseitige Knöchelstütze nach Anspruch 1, bei der das dritte Riementeil (50) konfiguriert ist, durch eines der Paare von Schlitzen zu passen, ein erstes Mal eine Rückseite der Knöchelstütze (110) zu kreuzen und ein zweites Mal eine Vorderseite der Knöchelstütze (110) zu kreuzen.

6. Doppelseitige Knöchelstütze nach Anspruch 1, bei der das dritte Riementeil (50) ein erstes Ende (52) und ein zweites Ende (54) hat, wobei das erste (52) und das zweite (54) Ende gestaltet sind, lösbar an dem ersten horizontalen Riemen (42) nach dem erstmaligen Kreuzen hinter der Knöchelstütze (110) und dem zweitmaligen Kreuzen vor der Knöchelstütze (110) befestigbar zu sein.

7. Doppelseitige Knöchelstütze nach einem der Ansprüche 1 bis 6, bei der die erste Seitenplatte (22) an dem ersten Bügelarm (118) an dem ersten Schwenkpunkt (126) so befestigt ist, dass die erste Seitenplatte (22) zu einer Vorderseite des Bügels (114) und zu einer Hinterseite des Bügels (114) schwenken kann, aber in der Bewegung von Seite zu Seite gehemmt ist.

8. Doppelseitige Knöchelstütze nach einem der Ansprüche 1 bis 7, bei der die zweite Seitenplatte (24) an dem zweiten Bügelarm (120) an dem zweiten Schwenkpunkt (128) derart befestigt ist, dass die zweite Seitenplatte (24) zu einer Vorderseite des Bügels (114) und zu einer Hinterseite des Bügels (114) schwenken kann, aber in der Bewegung von Seite zu Seite gehemmt ist.

9. Doppelseitige Knöchelstütze nach einem der Ansprüche 1 bis 8, bei der die erste Seitenplatte (22) zu einer vertikalen Mittellinie (10A, 11A) symmetrisch ist.

10. Doppelseitige Knöchelstütze nach einem der Ansprüche 1 bis 9, bei der die zweite Seitenplatte (24) zu einer vertikalen Mittellinie (10A, 11A) symmetrisch ist.

11. Doppelseitige Knöchelstütze nach einem der Ansprüche 1 bis 10, bei der der Bügel (114) zu einer Ebene, die durch den ersten Schwenkpunkt (126) und den zweiten Schwenkpunkt (128) gezogen ist und die senkrecht zu der Basisplatte (116) ist, symmetrisch ist.

## Revendications

1. Attelle réversible pour cheville (110) qui est adaptée de manière à pouvoir être portée de façon réversible sur une cheville gauche ou sur une cheville droite, l'attelle pour cheville (110) comprenant :
un étrier (114) qui inclut une plaque de base (116), une première branche d'étrier (118) et une seconde branche d'étrier (120) ;
une première plaque latérale (22) qui est fixée à la première branche d'étrier (118) au niveau d'un premier point de pivot (126), la première plaque latérale (22) incluant une première surface interne (22A) et une première surface externe (22B), une première fente allongée (46) étant formée dans la première surface externe (22B) ;
une seconde plaque latérale (24) qui est fixée à la seconde branche d'étrier (120) au niveau d'un second point de pivot (128), la seconde plaque latérale (24) incluant une seconde surface interne (24A) et une seconde surface externe (24B), une seconde fente allongée (48) étant formée dans la seconde surface externe (24B) ;
un premier coussinet (34) qui peut être fixé de façon libérable à la première surface interne (22A) ;
un second coussinet (36) qui peut être fixé de façon libérable à la seconde surface interne (24A) ;
une première sangle horizontale (42) qui peut être fixée de façon libérable autour d'un extérieur du premier coussinet (34) et du second coussinet (36), la première sangle horizontale (42) fixant de façon conjointe une partie supérieure de l'attelle pour cheville (110) ;
une deuxième sangle horizontale (44) qui s'étend au travers de la première fente allongée (46) et de la seconde fente allongée (48), la deuxième sangle horizontale (44) pouvant être fixée de façon libérable à elle-même, la deuxième sangle horizontale (44) fixant de façon conjointe une partie intermédiaire (38) de l'attelle pour cheville (110) ; et
un troisième élément de sangle (50), dans laquelle :
la plaque de base (116) inclut une première paire (150) de fentes (154, 156) pour recevoir le troisième élément de sangle (50) lorsque l'attelle pour cheville (110) est configurée pour une utilisation sur une cheville droite et une seconde paire (152) de fentes (158, 160) pour recevoir le troisième élément de sangle (50) lorsque l'attelle pour cheville (110) est configurée pour une utilisation sur une cheville gauche.

2. Attelle réversible pour cheville selon la revendication 1, dans laquelle l'étrier (114) comporte un premier côté et un second côté, la première branche d'étrier (118) étant approximativement centrée le long du premier côté et la seconde branche d'étrier (120) étant approximativement centrée le long du second côté.

3. Attelle réversible pour cheville selon la revendication 1 ou la revendication 2, dans laquelle la plaque de base (116), la première branche d'étrier (118) et la seconde branche d'étrier (120) sont moulées d'un seul tenant en tant que pièce unitaire.

4. Attelle réversible pour cheville selon l'une quelconque des revendications 1 à 3, comprenant en outre un coussinet en mousse (16A) qui est adapté de manière à être ajusté sur la plaque de base (116).

5. Attelle réversible pour cheville selon la revendication 1, dans laquelle le troisième élément de sangle (50) est configuré de manière à être ajusté au travers d'une fente de la paire de fentes, de manière à réaliser un croisement une première fois sur un côté arrière de l'attelle pour cheville (110) et de manière à réaliser un croisement une seconde fois sur un côté avant de l'attelle pour cheville (110).

6. Attelle réversible pour cheville selon la revendication 1, dans laquelle le troisième élément de sangle (50) comporte une première extrémité (52) et une seconde extrémité (54), et les première (52) et seconde (54) extrémités sont adaptées de manière à pouvoir être fixées de façon libérable sur la première sangle horizontale (42) après croisement la première fois derrière l'attelle pour cheville (110) et après croisement la seconde fois à l'avant de l'attelle pour cheville (110).

7. Attelle réversible pour cheville selon l'une quelconque des revendications 1 à 6, dans laquelle la première plaque latérale (22) est fixée à la première branche d'étrier (118) au niveau du premier point de pivot (126) de telle sorte que la première plaque latérale (22) puisse pivoter en direction d'un avant de l'étrier (114) et en direction d'un arrière de l'étrier (114) mais de telle sorte qu'elle soit contrainte de manière à ne pas pouvoir se déplacer latéralement.

8. Attelle réversible pour cheville selon l'une quelconque des revendications 1 à 7, dans laquelle la seconde plaque latérale (24) est fixée à la seconde branche d'étrier (120) au niveau du second point de pivot (128) de telle sorte que la seconde plaque latérale (24) puisse pivoter en direction d'un avant de l'étrier (114) et en direction d'un arrière de l'étrier (114) mais de telle sorte qu'elle soit contrainte de manière à ne pas pouvoir se déplacer latéralement.

9. Attelle réversible pour cheville selon l'une quelconque des revendications 1 à 8, dans laquelle la première plaque latérale (22) est symétrique par rapport à une ligne médiane verticale (10A, 11A).

10. Attelle réversible pour cheville selon l'une quelconque des revendications 1 à 9, dans laquelle la seconde plaque latérale (24) est symétrique par rapport à une ligne médiane verticale (10A, 11A).

11. Attelle réversible pour cheville selon l'une quelconque des revendications 1 à 10, dans laquelle l'étrier (114) est symétrique par rapport à un plan qui est tracé de manière à passer par le premier point de pivot (126) et par le second point de pivot (128) et qui est perpendiculaire à la plaque de base (116).
